(19) Europäisches Patentamt
European Patent Office
Office européen des brevets

(11) Veröffentlichungsnummer: **0 401 625 B1**

(12) # EUROPÄISCHE PATENTSCHRIFT

(45) Veröffentlichungstag der Patentschrift: **29.06.94**

(51) Int. Cl.⁵: **C08F 226/00**, C08F 222/00, C08L 63/00, A61K 9/20, A61K 47/48

(21) Anmeldenummer: **90110028.9**

(22) Anmeldetag: **26.05.90**

Die Akte enthält technische Angaben, die nach dem Eingang der Anmeldung eingereicht wurden und die nicht in dieser Patentschrift enthalten sind.

(54) **Alternierende Copolymere, Verfahren zu ihrer Herstellung sowie deren Verwendung.**

(30) Priorität: **09.06.89 DE 3918893**

(43) Veröffentlichungstag der Anmeldung:
**12.12.90 Patentblatt 90/50**

(45) Bekanntmachung des Hinweises auf die Patenterteilung:
**29.06.94 Patentblatt 94/26**

(84) Benannte Vertragsstaaten:
**AT BE CH DE ES FR GB IT LI NL SE**

(56) Entgegenhaltungen:
**EP-A- 0 113 061**
**US-A- 2 606 892**

(73) Patentinhaber: **BAYER AG**

**D-51368 Leverkusen(DE)**

(72) Erfinder: **Mormann, Werner, Prof. Dr.**
**Zum Wolfsloch 30**
**D-5910 Kreuztal(DE)**
Erfinder: **Schmalz, Kerstin**
**Schubertstrasse 2**
**D-5900 Siegen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft alternierende Copolymere aus Alkenylisocyanaten und elektronenarmen Olefinen, ein Verfahren zu deren Herstellung sowie deren Verwendung.

In EP-A 113 061 werden statistische Copolymere beschrieben, die einpolymerisiert ein N(1-alkenyl)-isocyanat und einen Ester der Acrylsäure oder Methacrylsäure enthalten. Das Copolymerisat weist ein mittleres Molekulargewicht von 500 bis 10 000 auf.

Alternierende Copolymere von Alkenylisocyanaten und elektronenarmen Olefinen sind in der Literatur bisher nicht beschrieben worden. Die Homopolymerisation von höheren Alkenylisocyanaten ergibt lediglich Polymere mit niedrigem Polymerisationsgrad.

Aufgabe war es, Polymere aus Alkenylisocyanaten mit hohem Polymerisationsgrad zur Verfügung zu stellen, die gegebenenfalls unterschiedlich reaktive Gruppen aufweisen, welche unabhängig voneinander in Reaktion treten können.

Gegenstand der Erfindung sind alternierende Copolymere

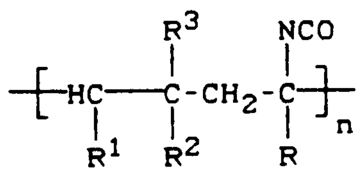

oder

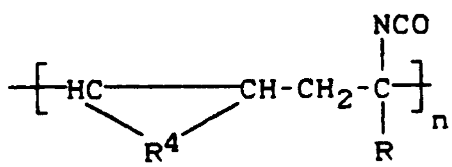

mit n = 10-5000
bestehend aus

a) Alkenylisocyanaten der Formel

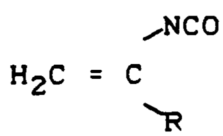 ,

wobei

R = Wasserstoff, einen Alkylrest mit 1 bis 6 Kohlenstoffatomen oder einen Alkancarbonsäurealkylesterrest bedeutet, wobei die Alkankette aus 1 bis 6 Kohlenstoffatomen und der Alkylester aus 1 bis 6 Kohlenstoffatomen besteht, und

b) elektronenarmen Olefinen der Formel

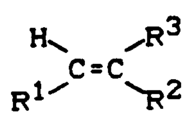

oder

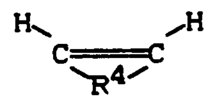

wobei

$R^1$ = H, $R^2$ = CN oder $COOR^5$ und $R^3$ = CN oder $COOR^5$

oder

$R^1$ = CN oder $COOR^5$, $R^2$ = CN oder $COOR^5$ und $R^3$ = H

oder

$R^1$ = CN oder $COOR^5$, $R^2$ = H und $R^3$ = CN oder $COOR^5$

und

$R^4$ = (O)C-O-C(O) oder (O)C-N(H)-C(O)

und

$R^5$ = Alkylrest mit 1 bis 8 Kohlenstoffatomen

bedeuten.

Besonders bevorzugt sind alternierende Copolymere, bei denen als Alkenylisocyanatkomponente Isopropenylisocyanat, Vinylisocyanat oder 4-Isocyanato-4-pentensäurealkylester eingesetzt werden.

Alternierende Copolymere mit Maleinsäureanhydrid, Maleinsäureimid, Fumarsäuredinitril, Maleinsäuredinitril, Maleinsäureester, Fumarsäureester oder Cyanacrylat als elektronenarme Olefinkomponente sind bevorzugte Ausführungsformen.

Ganz besonders bevorzugt sind Copolymere der oben genannten ungesättigten Isocyanate mit Maleinsäureanhydrid.

Ein weiterer Gegenstand der Erfindung ist ein Verfahren zur Herstellung dieser alternierenden Copolymeren, wobei bei einer Temperatur zwischen 0 und 100 ° C Alkenylisocyanate der Formel

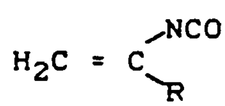

mit

R = H, Alkylrest mit 1 bis 6 Kohlenstoffatomen oder Alkancarbonsäurealkylesterrest mit einer Alkankette und einem Alkylester aus 1 bis 4 Kohlenstoffatomen mit elektronenarmen Olefinen der Formel

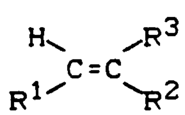

oder

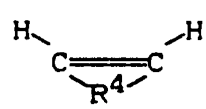

mit

$R^1$ = H, $R^2$ = CN oder $COOR^5$ und $R^3$ = CN oder $COOR^5$

oder

$R^1$ = CN oder $COOR^5$, $R^2$ = CN oder $COOR^5$ und $R^3$ = H
oder
$R^1$ = CN oder $COOR^5$, $R^2$ = H und $R^3$ = CN oder $COOR^5$
und
$R^4$ = (O)-C-OC(O) oder (O)C-N(H)-C(O)
und
$R^5$ = Alkylrest mit 1 bis 8 Kohlenstoffatomen
unter Zusatz von Initiatoren copolymerisiert werden.

Bevorzugt wird die Copolymerisation in Substanz, in Lösung oder als Fällungspolymerisation durchgeführt.

In einer besonderen Ausführungsform wird die Copolymerisation bei Temperaturen zwischen 0° C und 80° C durchgeführt.

Als Initiatoren werden vorzugsweise aliphatische Azoverbindungen, Peroxide oder Redoxinitiatoren eingesetzt.

Diese alternierenden Copolymere können u.a. als Vernetzer für Epoxide, Polyhydroxyverbindungen und Diamine verwendet werden.

Des weiteren können die alternierenden Copolymere als Träger für Arzneimittel verwendet werden.

Die Copolymerisation z.B. von Isopropenylisocyanat, von 4-Isocyanato-4-pentensäuremethylester oder von Vinylisocyanat mit Maleinsäureanhydrid führt in hohen Ausbeuten zu alternierenden Copolymeren mit hohen reduzierten Viskositäten. Das molare Verhältnis der Monomeren im Reaktionsgemisch kann dabei in weitem Rahmen, z.B. von 1:5 bis 5:1 variiert und die Reaktion bis zu hohen Umsätzen (d.h. fast vollständigen Einbau des in geringerer Menge vorhandenen Monomeren) geführt werden, ohne daß die alternierende Sequenz im Polymeren beeiflußt wird. Bevorzugt ist die Copolymerisation in äquimolarem Verhältnis der beiden Monomeren. Die Polymerisation kann z.B. in Substanz, in Lösung oder als Fällungspolymerisation aus einer 10 %igen Lösung in Benzol durchgeführt werden.

Die beiden reaktiven Gruppen der ungesättigten Monomeren sind unter den Reaktionsbedingungen und in den isolierten Polymeren stabil. Die ist insofern überraschend, als zu erwarten war, daß die bekannte Reaktion zwischen Isocyanat- und Anhydridgruppe wenigstens zu einem gewissen Anteil stattfindet, was vernetzte Produkte zur Folge gehabt hätte.

Die Erfindung soll anhand der folgenden Beispiele näher erläutert werden.

Beispiel 1

Synthese von alternierenden Copolymeren aus Isopropenylisocyanat und Maleinsäureanhydrid durch Fällungspolymerisation

In einem im Vakuum ausgeheizten und unter Argon abgekühlten 50 ml Kolben werden 20 ml Benzol, 1,04 g (12,5 mmol) Isopropenylisocyanat, 1,19 g (12,5 mmol) Maleinsäureanhydrid und 20,4 mg (0,12 mol) Azodiisobuttersäurenitril 10 Stunden bei 60° C gerührt. Aus der schwach gelb gefärbten Lösung beginnt nach etwa einer Stunde das Polymer auszufallen. Nach zehn Stunden saugt man den Niederschlag über eine Schutzgasfritte ab, wäscht mit Benzol nach und trocknet bei 40° C im Ölpumpenvakuum.

| | |
|---|---|
| Ausbeute: | 1,96 g (88 %) |
| IR (Film aus Aceton): | 2270, 1850 und 1780 $cm^{-1}$ |
| Elementaranalyse: | C, H, N Abweichung vom berechneten Wert < 0,1 % |
| Inhärente Viskosität: | 0,44 dl/g gemessen in Aceton bei 0,264 g/dl |

Beispiele 2-10

Synthese von alternierenden Copolymeren (allgemeine Arbeitsvorschrift)

Man verfährt wie unter Beispiel 1 beschrieben. Die Comonomeren und Azoisobutyronitril (AIBN), 0,5 Mol%, bezogen auf die maximal polymerisierbare Substanz werden im angegebenen Molverhältnis (Tabellen 1-4) eingewogen und in Benzol gelöst, die Lösung wird entgast und bei 60° C polymerisiert. Nach der in den Tabellen angegebenen Zeit wird das ausgefallene Polymer in einer Schutzgasfritte abgesaugt. Das Produkt wird nochmals in Benzol ausgerührt und anschließend im Ölpumpenvakuum getrocknet.

Allgemeine Eigenschaften der synthetisierten Polymere:

Löslichkeit: Die Polymere sind in Aceton, in 2-Butanon (Methylethylketon), N,N-Dimethylformamid und N,N-Dimethylacetamid löslich.

IR-Spektren: Aus einer Acetonlösung werden klare Filme der Polymeren erhalten, die sich bei längerem Stehen an der Luft (15-30 min) trüben. Die IR-Spektren dieser Filme zeigen die Banden der jeweils enthaltenen charakteristischen Strukturelemente:

| | |
|---|---|
| Isocyanat | 2270 $cm^{-1}$ |
| Methylester | 1740 $cm^{-1}$ |
| Anhydrid | 1780 $cm^{-1}$, 1850 $cm^{-1}$ |
| Nitril | 2270 $cm^{-1}$ (überdeckt vom Isocyanat). |

**Tabelle 1:** Zusammensetzung und Eigenschaften von alternierenden Copolymeren aus 2-Isocyanatopropen und Maleinsäureanhydrid

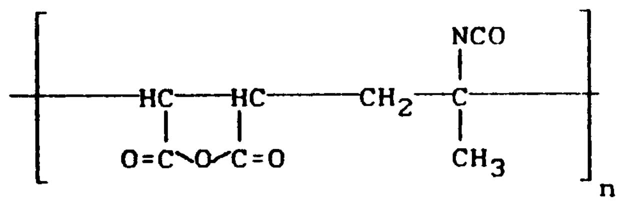

| Nr. | Molver-hältnis[a)] | Reaktionszeit [h] | Ausbeute[b)] [%] | η [dl/g] | $^1$H-NMR [ppm] |
|---|---|---|---|---|---|
| 1 | 1 : 1 | 8 | 88 | 0,44 | 3,8 (CH, Anhydrid); |
| 2 | 1 : 3 | 15 | 80 | 0,31 | 2,8 ($CH_2$); |
| 3 | 3 : 1 | 15 | 71 | 0,30 | 2,0 ($CH_3$)[c)]; |
| 4 | 1 : 2 | 24 | 90 | 0,26 | |
| 5 | 2 : 1 | 24 | 93 | 0,43 | |

a) Isocyanat: Maleinsäureanhydrid
b) bezogen auf den vollständigen Umsatz der im Unterschuß vorliegenden Komponente
c) von Aceton (Lösungsmittel) teilweise überlagert
η inhärente Viskosität

**T a b e l l e  2:** Zusammensetzung und Eigenschaften von alternierenden Copolymeren aus 4-Isocyanato-4-pentensäuremethylester und Maleinsäureanhydrid

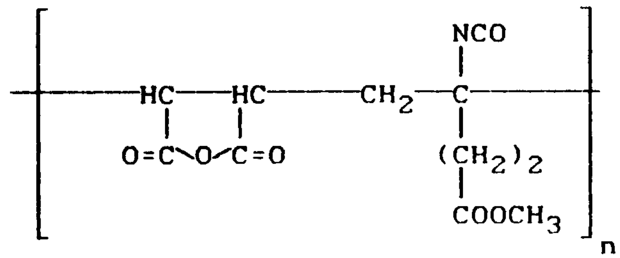

| Nr. | Molver-hältnis[a)] | Reaktionszeit [h] | Ausbeute[b)] [%] | η [dl/g] | $^1$H-NMR [ppm] |
|---|---|---|---|---|---|
| 6 | 1 : 1 | 42 | 29 | 0,17 | 3,7 (CH, Anhydrid); |
| 7 | 1 : 3 | 42 | 25 | 0,18 | 3,6 ($CH_3$); 2,6 ($CH_2$) |
| 8 | 3 : 1 | 42 | 15 | 0,17 | |

a) Isocyanat: Maleinsäureanhydrid
b) bezogen auf den vollständigen Umsatz der im Unterschuß vorliegenden Komponente
η inhärente Viskosität

**T a b e l l e 3:** Zusammensetzung und Eigenschaften von alternierenden Copolymeren aus Isocyanatoethen und Maleinsäureanhydrid

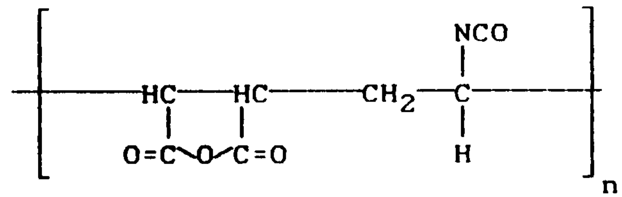

| Nr. | Molver-[a)] hältnis | Reaktionszeit [h] | Ausbeute[b)] [%] | η [dl/g] | $^1$H-NMR [ppm] |
|---|---|---|---|---|---|
| 9 | 1 : 1 | 15 | 93 | 0,17 | 4,7 (CH, Isocyanat); |
| 7 | 1 : 3 | 42 | 25 | 0,18 | 3,6 (CH, Anhydrid); |
| 8 | 3 : 1 | 42 | 15 | 0,17 | 2,5 ($CH_2$) |

a) Isocyanat: Maleinsäureanhydrid
b) bezogen auf vollständigen Umsatz der im Unterschuß vorliegenden Komponente
η inhärente Viskosität

Tabelle 4: Zusammensetzung und Eigenschaften von alternierenden Copolymeren aus Isocyanatopropen und Fumarsäuredinitril

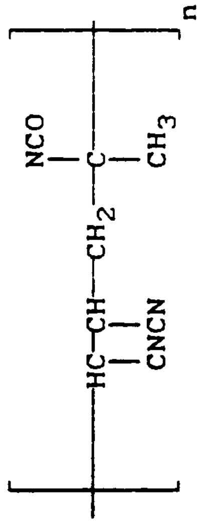

| Nr. | Molverhältnis[a] | Reaktionszeit [h] | Ausbeute[b] [%] | η [dl/g] | $^1$H-NMR [ppm] |
|---|---|---|---|---|---|
| 10 | 1 : 1 | 24 | 31 | 0,14 | 3,9 (CH); 2,5 ($CH_2$); 2,0 ($CH_3$)[c]; |

a) Isocyanat: Fumarsäuredinitril
b) bezogen auf vollständigen Umsatz der im Unterschuß vorliegenden Komponente
c) von Aceton (Lösungsmittel) teilweise überlagert
η inhärente Viskosität

## Patentansprüche

**1.** Alternierende Copolymere

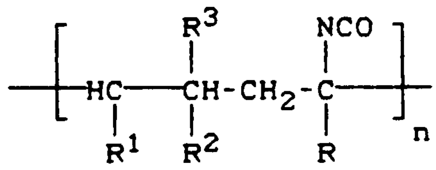

oder

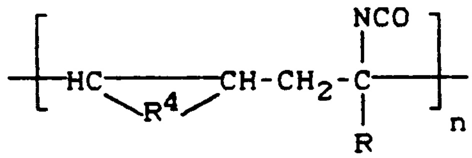

mit n = 10-5000 bestehend aus den Einheiten

a) Alkenylisocyanate folgender Formel

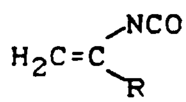

und

b) elektronenarme Olefine

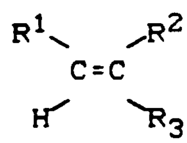

oder

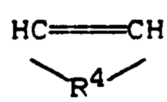

wobei

$R^1$ = H, $R^2$ = CN oder $COOR^5$ und $R^3$ = CN oder $COOR^5$

oder

$R^1$ = CN oder $COOR^5$, $R^2$ = CN oder $COOR^5$ und $R^3$ = H

oder

$R^1$ = CN oder $COOR^5$, $R^2$ = H und $R^3$ = CN oder $COOR^5$

und

$R^4$ = (O)C-O-C(O) oder (O)C-N(H)-C(O)

und

$R^5$ = Alkylrest mit 1 bis 8 Kohlenstoffatomen

und

R = Wasserstoff, Alkylrest mit 1 bis 6 Kohlenstoffatomen oder Alkancarbonsäurealkylesterrest mit Alkankette aus 1 bis 6 Kohlenstoffatomen und einem Alkylester aus 1 bis 6 Kohlenstoffatomen bedeuten.

**2.** Alternierende Copolymere nach Anspruch 1, dadurch gekennzeichnet, daß das Alkenylisocyanat Vinylisocyanat, Isopropenylisocyanat oder 4-Isocyanato-4-pentensäurealkylester ist.

**3.** Alternierende Copolymere nach Anspruch 1 oder 2, dadurch gekennzeichnet, daß das elektronenarme Olefin Maleinsäureanhydrid, Maleinsäureimid, Fumarsäuredinitril, Maleinsäuredinitril, Maleinsäureester, Fumarsäureester oder Cyanacrylat ist.

**4.** Verfahren zur Herstellung von alternierenden Copolymeren gemäß den Ansprüchen 1 bis 3, dadurch gekennzeichnet, daß bei einer Temperatur zwischen 0 und 100° C Alkenylisocyanate der Formel

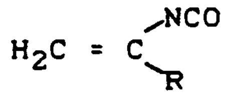

mit

R = H, Alkylrest mit 1 - 6 C-Atomen, Alkancarbonsäure-alkylesterrest mit 1-6 C-Atomen im Alkanrest und im Alkylrest

mit

elektronenarmen Olefinen der Formel

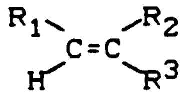

oder

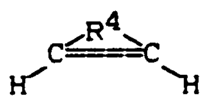

mit

$R^1$ = H, $R^2$ = CN oder $COOR^5$ und $R^3$ = CN oder $COOR^5$ oder
$R^1$ = CN oder $COOR^5$, $R^2$ = CN oder $COOR^5$ und $R^3$ = H oder
$R^1$ = CN oder $COOR^5$, $R^2$ = H und $R^3$ = CN oder $COOR^5$ und
$R^4$ = (O)-C-OC(O) oder (O)C-N(H)-C(O) und
$R^5$ = Alkylrest mit 1 bis 8 Kohlenstoffatomen

unter Zusatz von Initiatoren copolymerisiert werden.

**5.** Verfahren gemäß Anspruch 4, dadurch gekennzeichnet, daß die Copolymerisation in Substanz, in Lösung oder als Fällungspolymerisation durchgeführt wird.

**6.** Verfahren gemäß einem der Ansprüche 4 oder 5, dadurch gekennzeichnet, daß die Copolymerisation bei Temperaturen zwischen 0 und 80° C durchgeführt wird.

**7.** Verfahren gemäß einem der Ansprüche 4 bis 6, dadurch gekennzeichnet, daß als Initiatoren aliphatische Azoverbindungen, Peroxide oder Redoxinitiatoren eingesetzt werden.

**8.** Verwendung der alternierenden Copolymere gemäß einem der Ansprüche 1 bis 3 als Vernetzer für Epoxide, Polyhydroxyverbindungen oder Diamine.

**9.** Verwendung der alternierenden Copolymere gemäß einem der Ansprüch 1 bis 3 als Träger für Arzneimittel.

## Claims

**1.** Alternating copolymers

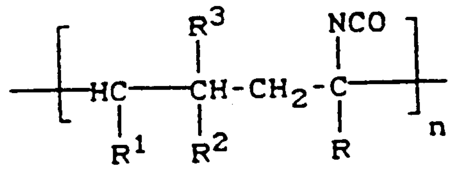

or

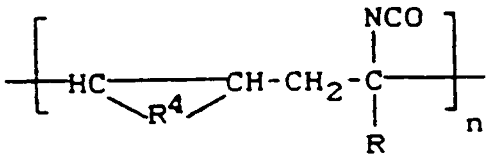

with n = 10-5000, consisting of the units

a) alkenyl isocyanates corresponding to the following formula

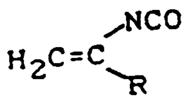

and

b) olefins poor in electrons

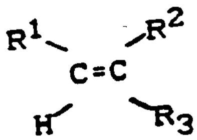

or

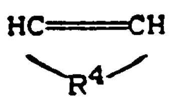

where

$R^1$ = H, $R^2$ = CN or $COOR^5$ and $R^3$ = CN or $COOR^5$

or

$R^1$ = CN or $COOR^5$, $R^2$ = CN or $COOR^5$ and $R^3$ = H

or

$R^1$ = CN or $COOR^5$, $R^2$ = H and $R^3$ = CN or $COOR^5$

and

$R^4$ = (O)C-O-C(O) or (O)C-N(H)-C(O)

and

$R^5$ = $C_{1-8}$ alkyl

and

R = hydrogen, a $C_{1-6}$ alkyl radical or an alkane carboxylic acid alkyl ester group with a $C_{1-6}$ alkyl chain and a $C_{1-6}$ alkyl ester.

2. Alternating copolymers as claimed in claim 1, characterized in that the alkenyl isocyanate is vinyl isocyanate, isopropenyl isocyanate or 4-isocyanato-4-pentenoic acid alkyl ester.

3. Alternating copolymers as claimed in claim 1 or 2, characterized in that the olefin low in electrons is maleic anhydride, maleic acid imide, fumaric acid dinitrile, maleic acid dinitrile, maleic acid ester, fumaric acid ester or cyanoacrylate.

4. A process for the production of the alternating copolymers claimed in claims 1 to 3, characterized in that alkenyl isocyanates corresponding to the following formula

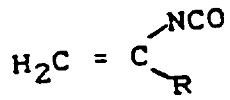

with

R = H, $C_{1-6}$ alkyl, an alkane carboxylic acid alkyl ester group with 1 to 6 carbon atoms in the alkane component and in the alkyl component,

are copolymerized at 0 to 100 ° C with olefins poor in electrons corresponding to the following formula

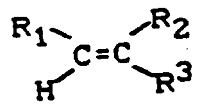

or

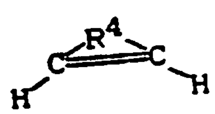

where

$R^1$ = H, $R^2$ = CN or $COOR^5$ and $R^3$ = CN or $COOR^5$

or

$R^1$ = CN or $COOR^5$, $R^2$ = CN or $COOR^5$ and $R^3$ = H

or

$R^1$ = CN or $COOR^5$, $R^2$ = H and $R^3$ = CN or $COOR^5$

and

$R^4$ = (O)-C-OC(O) or (O)C-N(H)-C(O)

and

$R^5$ = $C_{1-8}$ alkyl,

with addition of initiators.

5. A process as claimed in claim 4, characterized in that the copolymerization is carried out in bulk, in solution or as precipitation polymerization.

6. A process as claimed in claim 4 or 5, characterized in that the copolymerization is carried out at 0 to 80 ° C.

7. A process as claimed in any of claims 4 to 6, characterized in that aliphatic azo compounds, peroxides or redox initiators are used as the initiators.

8. The use of the alternating copolymers claimed in any of claims 1 to 3 as crosslinking agents for epoxides, polyhydroxy compounds or diamines.

**9.** The use of the alternating copolymers claimed in any of claims 1 to 3 as supports for medicaments.

**Revendications**

**1.** Copolymères alternés

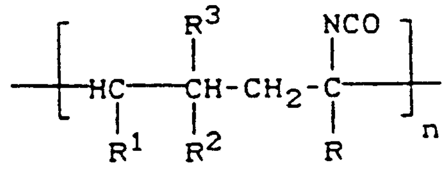

ou

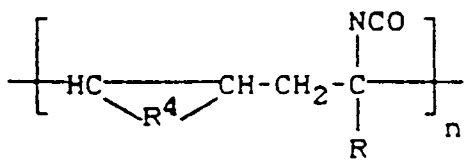

avec n = 10-5000, constitués des motifs

a) alcénylisocyanates de formule suivante :

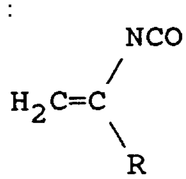

et

b) oléfines pauvres en électrons

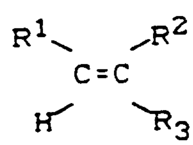

ou

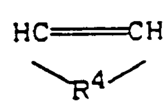

où

$R^1$ = H, $R^2$ = CN ou $COOR^5$ et $R^3$ = CN ou $COOR^5$,

ou bien

$R^1$ = CN ou $COOR^5$, $R^2$ = CN ou $COOR^5$ et $R^3$ = H,

ou bien

$R^1$ = CN ou $COOR^5$, $R^2$ = H et $R^3$ = CN ou $COOR^5$

et

$R^4$ = (O)C-O-C(O) ou (O)C-N(H)-C(O)

$R^5$ = reste alkyle ayant 1 à 8 atomes de carbone

et

R = hydrogène, reste alkyle ayant 1 à 6 atomes de carbone ou reste ester alkylique d'acide alcane-carboxylique avec une chaîne d'alcane formée de 1 à 6 atomes de carbone et un ester alkylique formé de 1 à 6 atomes de carbone.

**2.** Copolymères alternés suivant la revendication 1, caractérisés en ce que l'alcénylisocyanate est le vinylisocyanate, l'isopropénylisocyanate ou un ester alkylique d'acide 4-isocyanato-4-penténoïque.

**3.** Copolymères alternés suivant la revendication 1 ou 2, caractérisés en ce que l'oléfine pauvre en électrons est l'anhydride d'acide maléique, l'imide d'acide maléique, le dinitrile d'acide fumarique, le dinitrile d'acide maléique, un ester d'acide maléique, un ester d'acide fumarique ou un cyanacrylate.

**4.** Procédé de production de copolymères alternés suivant les revendications 1 à 3, caractérisé en ce qu'on copolymérise, avec addition d'initiateurs, à une température comprise entre 0 et 100 °C, des alcénylisocyanates de formule

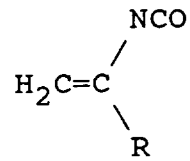

où

R = H, reste alkyle ayant 1 à 6 atomes de carbone, reste ester d'alkyle d'acide alcane-carboxylique ayant 1 à 6 atomes de carbone dans le reste alcane et dans le reste alkyle avec

des oléfines pauvres en électrons, de formule

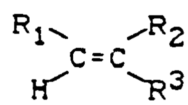

ou

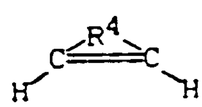

où

$R^1$ = H, $R^2$ = CN ou $COOR^5$ et $R^3$ = CN ou $COOR^5$,

ou bien

$R^1$ = CN ou $COOR^5$, $R^2$ = CN ou $COOR^5$ et $R^3$ = H,

ou bien

$R^1$ = CN ou $COOR^5$, $R^2$ = H et $R^3$ = CN ou $COOR^5$

et

$R^4$ = (O)-C-OC(O) ou (O)C-N(H)-C(O)

et

$R^5$ = reste alkyle ayant 1 à 8 atomes de carbone.

**5.** Procédé suivant la revendication 4, caractérisé en ce que la copolymérisation est conduite en substance, en solution ou comme polymérisation avec précipitation.

**6.** Procédé suivant l'une des revendications 4 ou 5, caractérisé en ce que la copolymérisation est conduite à des températures comprises entre 0 et 80 °C.

7. Procédé suivant l'une des revendications 4 à 6, caractérisé en ce qu'on utilise comme initiateurs des composés azoïques aliphatiques, des peroxydes ou des initiateurs redox.

8. Utilisation des copolymères alternés suivant l'une des revendications 1 à 3 comme agents réticulants pour époxydes, composés polyhydroxyliques ou diamines.

9. Utilisation des copolymères alternés suivant l'une des revendications 1 à 3 comme support pour médicaments.